# EUROPÄISCHE PATENTSCHRIFT

(11) **EP 2 882 356 B1**
(45) Veröffentlichungstag und Bekanntmachung des Hinweises auf die Patenterteilung: **06.09.2017**
(21) Anmeldenummer: 13741737.4
(22) Anmeldetag: 24.07.2013
(51) Int. Cl.: A61B 17/16

(54) **PNEUMATISCH BETÄTIGBARES CHIRURGISCHES INSTRUMENT**
PNEUMATICALLY CONTROLLABLE SURGICAL INSTRUMENT
INSTRUMENT CHIRURGICAL PNEUMATIQUE

(30) Priorität: 08.08.2012 DE 102012107292
(43) Veröffentlichungstag der Anmeldung: 17.06.2015
(73) Patentinhaber: Aesculap AG, 78532 Tuttlingen (DE)
(72) Erfinder: SCHOLTEN, Thomas, 78532 Tuttlingen (DE); WEISSHAUPT, Dieter, 78194 Immendingen (DE); NESPER, Markus, 78532 Tuttlingen (DE); SCHULZ, Peter, 79843 Löffingen (DE)
(74) Vertreter: Hoeger, Stellrecht & Partner Patentanwälte mbB
(86) Internationale Anmeldenummer: PCT/EP2013/065615
(87) Internationale Veröffentlichungsnummer: WO 2014/023575

(56) Entgegenhaltungen:
- WO-A1-2009/109198
- DE-A1-102010 012 011
- DE-U1-202007 006 801
- US-A1- 2006 151 567

## Beschreibung

Die Erfindung betrifft ein pneumatisch betätigbares chirurgisches Instrument umfassend einen Handgriff, ein an dem Handgriff angeordnetes druckgasbetriebenes Werkzeug, einen austauschbaren Druckgasspeicher sowie eine Verriegelungsvorrichtung zum Festlegen des Druckgasspeichers an dem Handgriff in einer Arbeitsstellung, in welcher der Druckgasspeicher an einen Druckgaskanal angeschlossen ist, mit dem das Werkzeug mit Druckgas versorgt wird.

Die eingangs beschriebenen pneumatisch betätigbaren chirurgischen Instrumente werden vielfach mit Druckgasspeichern in Form von Druckgaspatronen mit Druckgas versorgt, so dass eine Verbindung des Instruments mit einem Druckgasschlauch zur Versorgung mit Druckgas entfallen kann und eine maximale Handlichkeit des chirurgischen Instruments erzielt wird.

Problematisch ist bei den pneumatisch betriebenen Instrumenten, die mit einem Druckgasspeicher ausgerüstet sind, dass am Ende der Nutzungsdauer eines Druckgasspeichers immer ein Restvolumen an Druckgas enthalten ist, das beim Entnehmen des Druckgasspeichers aus dem Instrument mit einem lauten Knall entweicht.

Erfindungsgemäße pneumatisch betriebene Instrumente mit einem Druckgasspeicher kommen häufig bei diffizilen Eingriffen am zentralen Nervensystem zum Einsatz. Als Beispiel seien hier pneumatisch betriebene Knochenstanzen erwähnt, welche es erlauben, unter hohem Krafteinsatz Knochen- oder Gewebematerial am Patienten zu entnehmen.

Ist ein Operateur noch mit einem diffizilen Eingriff beschäftigt und im Hintergrund läuft der Austausch einer Druckgaspatrone eines pneumatisch betriebenen Instruments ab, kann es leicht zu einem Erschrecken des Operateurs und dadurch zu nicht kontrollierbaren Reaktionen kommen.

Bei pneumatisch betriebenen Knochenstanzen ist es insbesondere wichtig, dass die Stanzkraft während des Eingriffs am Patienten nicht nachlässt, da sonst beim Eingriff am Patienten der Schaft am Knochen fixiert bleiben kann, während andererseits die Kraft der Stanze zum Durchtrennen des Knochenmaterials nicht mehr ausreicht. Deswegen wird bei dieser Art von pneumatisch betriebenen Knochenstanzen die Funktion der Knochenstanze automatisch begrenzt, wenn ein Mindestdruck erreicht oder unterschritten wird.

Dadurch bleiben in dem Druckgasspeicher noch erhebliche Mengen an Druckgas übrig, beispielsweise beträgt hier der verbliebene Druckgasdruck ca. 2 bar, einen Druck, der ausreicht, bei jedem Wechsel des Druckgasspeichers einen mehr oder weniger lauten Knall zu erzeugen.

Andererseits kann es auch sein, dass, wenn nach Beendigung einer Operation von dem pneumatisch betriebenen Instrument und dem darin angeordneten Druckgasspeicher kein extensiver Gebrauch gemacht wurde, der Druckgasspeicher noch fast voll ist. Für eine weitere Verwendung des Instruments muss dieses aber dann wieder aufbereitet werden, was zwingend die Entfernung der Druckgaspatrone verlangt. Hier ist der Restdruck in dem Druckgasspeicher noch deutlich höher, und auch hier ist es wichtig, dass für das Personal beim Entfernen des Druckgasspeichers keine Gefahr besteht.

Aus der WO 2009/109198 A1 ist ein pneumatisch betätigbares Instrument mit einem austauschbaren Druckgasspeicher bekannt.

In der DE 10 2010 012 011 A1 wird ein pneumatisch betätigbares chirurgisches Instrument beschrieben, bei dem eine austauschbare Gaskartusche als Energiespeicher Verwendung findet. Fällt der Gasdruck in einer Versorgungsleitung erstmalig unter einen vorgegebenen Schwelldruck, der einem Restladezustand der Gaskartusche entspricht, so wird ein
Schaltelement von einer Feder in eine Position verschoben, in der die Versorgungsleitung mit einer Entlüftungsöffnung verbunden und die Kartusche drucklos gemacht wird.

Die DE 20 2007 006 801 U1 empfiehlt bei einem medizinischen Instrument mit einer austauschbaren Gaskartusche die Verwendung eines an der Gaskartusche festgelegten, mit der Gaskartusche austauschbaren Dichtelements.

Die US-Patentanmeldung US 2006/0151567 A1 beschreibt einen chirurgischen Klammerapplikator, der mit einer austauschbaren Gaskartusche betrieben werden kann.

Aufgabe der vorliegenden Erfindung ist es, das eingangs beschriebene pneumatisch betätigbare chirurgische Instrument in der Handhabung des Druckgasspeichers einfacher und sicherer zu machen.

Diese Aufgabe wird durch ein pneumatisch betätigbares chirurgisches Instrument mit den Merkmalen des Anspruchs 1 gelöst.

Ein wesentliches Element der vorliegenden Erfindung ist es, dass der Druckspeicher dem Instrument nicht ohne Weiteres entnommen werden kann, vielmehr muß zuerst die Entriegelungsvorrichtung betätigt werden, bevor dann in einem nachgelagerten Schritt, wenn die Festlegung des Druckgasspeichers in der Arbeitsstellung aufgehoben ist, der Druckgasspeicher gelöst und ausgetauscht werden kann. Erfindungsgemäss weist die Entriegelungsvorrichtung eine Entlüftungsvorrichtung auf, welche bei einer Betätigung der Entriegelungsvorrichtung aktiviert und aus einer Ruhestellung in eine erste Arbeitsstellung überführt wird, in der der Druckgaskanal mit einer Entlüftungsöffnung verbunden ist. Erfindungsgemäss ist ferner vorgesehen, dass die Entlüftungsvorrichtung in ihrer ersten Arbeitsstellung den Druckgasspeicher noch nicht freigibt sondern weiter blockiert, bis die Entriegelungsvorrichtung in eine zweite Arbeitsstellung bewegt wird, in der der Druckgaskanal immer noch mit der Entlüftungsöffnung verbunden ist. Der Druckgasspeicher wird erst in der zweiten Arbeitsstellung zum Trennen vom Instrument freigegeben.

Die Maßnahme ermöglicht, dass unabhängig vom verbleibenden Restdruck in dem Druckgasspeicher ein ausreichender Druckabbau, d.h. Entleerung des Druckgasspeichers, erfolgt, bevor die Entriegelungsvorrichtung in die zweite Arbeitsstellung überführt wird und der Druckgasspeicher von dem Instrument getrennt werden kann. Eine Fehlbedienung beim Wechseln des Druckgasspeichers wird somit verhindert.

Durch die Verwendung einer Entriegelungsvorrichtung, welche gemäss der vorliegenden Erfindung, eine Entlüftungsvorrichtung aufweist, wird ein kontrolliertes Entlüften des Druckgasspeichers möglich, so dass eine momentane, mit einem Knall verbundene Entlüftung des Druckgasspeichers unterbleibt.

Eine gewisse Abhilfe könnte bei mit einer Schraubverbindung ausgerüsteten Druckgasspeichern durch eine Verlängerung des Gewindes geschaffen werden, so dass eine Entnahme des Druckgasspeichers beispielsweise nur über mehrere 360°-Drehungen möglich ist, so dass während des Entfernens des Druckgasspeichers von dem chirurgischen Instrument, nach einer Trennung des Druckgasspeichers von dem Druckgaskanal des Instruments, ausreichend Zeit verbleibt, um den Druck abzubauen, solange der Druckgasspeicher mit seinem Gewindeabschnitt noch am Handgriff und dessen Gegengewinde im Eingriff gehalten ist.

Bei einer solchen Vorgehensweise ist der Anwender allerdings nicht gezwungen zunächst in einem vorgelagerten Schritt die Entriegelung vorzunehmen und erst dann den eigentlichen Schritt zum Austausch des Druckgasspeichers einzuleiten. Auch ist keine kontrollierte Entlüftung möglich, und ein Abschrauben des Druckgasspeichers von dem chirurgischen Instrument ließe sich auch nicht einfach handhaben. Insbesondere wäre der Austausch des Druckgasspeichers zeitaufwändig.

Demgegenüber kann das erfindungsgemäße pneumatisch betätigbare chirurgische Instrument schneller und in der Handhabung einfacher mit einer neuen Druckgaspatrone versorgt werden, und insbesondere lässt sich bei einer bevorzugten Ausführungsform, wie weiter unten noch beschrieben, das überschüssige Druckgas in einer vorgegebenen Richtung über eine Entlüftung aus dem Instrument ableiten.

Obwohl das erfindungsgemäße chirurgische Instrument verhindert, dass der Druckgasspeicher ohne kontrollierte Entlüftung von dem Instrument getrennt werden kann, lässt sich diese Funktion realisieren, ohne dass die Handhabung des Instruments komplizierter wird, insbesondere auch ohne dass das Instrument schwerer und ohne dass es teurer in der Herstellung wird.
Insbesondere lässt sich das pneumatisch betätigbare chirurgische Instrument auch mit einer Bajonett-Montage für den Druckgasspeicher kombinieren, bei der der Demontagevorgang aus einer simplen 120°-Drehung besteht und damit sehr schnell durchführbar ist, ohne dass dabei die Sicherheit beim Austausch des Druckgasspeichers beeinträchtigt wird.

Aus den eingangs genannten Gründen ist es vorteilhaft, wenn das erfindungsgemäße chirurgische Instrument mit einem Drucksensor zur Überwachung des Drucks in dem Druckgasspeicher ausgerüstet ist. Dies wird vorzugsweise mittels einer Überwachung des Betriebsdrucks in dem zum Werkzeug führenden Druckgaskanal realisiert.

Darüber hinaus ist es vorteilhaft, wenn das Instrument eine Anzeigevorrichtung für den aktuellen Füllzustand des Druckgasspeichers umfasst, die ebenfalls an den Betriebsdruck im Druckgaskanal gekoppelt sein kann, so dass der das Instrument verwendende Operateur jederzeit über den Füllzustand des Druckgasspeichers orientiert ist. Insbesondere kann ihm auf diese Weise dann angezeigt werden, wenn der Druckgasvorrat in dem Druckgasspeicher zu gering für eine weitere Nutzung des pneumatisch betriebenen Instruments ist, so dass er sofort den Grund für die blockierte Funktion des pneumatischen Instruments erkennen kann.

Bei einer besonders vorteilhaften Ausführungsform des pneumatisch betriebenen Instruments ist vorgesehen, dass die Verriegelungsvorrichtung und die Entriegelungsvorrichtung als eine mechanische Einheit ausgebildet sind. Dies vereinfacht das Einsetzen in und das Trennen des Druckgasspeichers von dem Instrument.

In diese mechanische Einheit aus Verriegelungsvorrichtung und Entriegelungsvorrichtung kann zusätzlich noch eine Anzeigevorrichtung für den aktuellen Füllzustand des Druckgasspeichers integriert werden, so dass alle drei Funktionen der Verriegelung, der Entriegelung und der Anzeige des Füllzustandes von einer mechanischen Einheit übernommen werden können. Dies hat fertigungstechnisch Vorteile, und darüber hinaus ist auch die Handhabung einfach und kann zusätzlich für eine sensorische Anzeige des aktuellen Füllzustands des Druckgasspeichers verwendet werden, so dass ohne Blickkontakt mit der Anzeigevorrichtung dem Operateur die Information über den aktuellen Füllzustand gegeben werden kann.

Bei einer besonders bevorzugten Ausführungsform des erfindungsgemäßen chirurgischen Instruments ist vorgesehen, dass die Entriegelungsvorrichtung mit einem einzigen Betätigungselement aus der Ruhestellung in die erste Arbeitsstellung und in die zweite Arbeitsstellung bewegbar ist.

Dies vereinfacht die Handhabung des chirurgischen Instruments bei dem Wechsel des Druckgasspeichers erheblich, wobei die Sicherheit für den Entriegelungsvorgang dadurch gegeben ist, dass das Betätigungselement über eine erste Arbeitsstellung, in der bereits eine Entlüftung stattfindet, in eine zweite Arbeitsstellung bewegt werden kann.

Der Anwender erhält über das bei der Entlüftung auftretende zischende Geräusch eine Rückmeldung über das Fortschreiten der Entleerung des Druckgasspeichers.

Weiter bevorzugt weist das erfindungsgemäße chirurgische Instrument einen Abluftkanal auf, der verbrauchtes Druckgas aus dem Werkzeug aufnimmt und in die Umgebung ableitet. Dies gilt zum Einen für den regulären Betrieb des chirurgischen Instruments und kann weiter bevorzugt auch für das Abführen des restlichen Druckgases aus dem Druckgasspeicher beim Wechsel desselben genutzt werden, indem die Entlüftungsöffnung mit dem Abluftkanal in Fluidverbindung steht bzw. im Zuge der Entriegelung in Fluidverbindung mit dem Abluftkanal gebracht wird.

Eine weitere Verminderung der Geräuschentwicklung bei einem Wechsel des Druckgasspeichers kann dadurch erreicht werden, dass entweder die Entlüftungsöffnung oder der Abluftkanal selbst mit einem Schalldämpfer versehen ist.

Bei einer weiter bevorzugten Ausführungsform des erfindungsgemäßen chirurgischen Instruments ist vorgesehen, dass die Verriegelungsvorrichtung pneumatisch, insbesondere durch das Druckgas des Druckgasspeichers, aktivierbar ist.

Damit wird die Verriegelungsvorrichtung erst aktiv, wenn der Druckgasspeicher mit der pneumatischen Einheit des Instruments in Fluidverbindung steht, und erst in diesem Zustand ist eine Verriegelung des Druckgasspeichers notwendig. Dies verhindert, dass bei einem ungünstigen Einsetzen des Druckgasspeichers, bei dem dieser gar nicht in Fluidverbindung mit der Pneumatikvorrichtung des Instruments gelangt, vorzeitig verriegelt wird und dann eine Korrektur der Position des eingebauten Druckgasspeichers verkompliziert wird.

Gemäß einer Variante des erfindungsgemäßen chirurgischen Instruments ist der Druckgasspeicher direkt am Handgriff des Instruments gehalten. Dies kann beispielsweise geschehen wie in der US 2006/0151567 A beschrieben.

Bei einer weiteren Alternative ist vorgesehen, dass der Druckgasspeicher in einem Gehäuse am Handgriff des Instruments gehalten ist. Beispiele hierfür finden sich in der DE 20 2007 006 801 U1 sowie auch in der US 2006/0151567 A. Hier wird das Gehäuse als Teil des Handgriffs ausgebildet. Erfindungsgemäß kann jedoch auch alternativ das Gehäuse als separates Teil ausgebildet und lösbar am Handgriff des Instruments befestigbar sein.

Weiter bevorzugt weisen erfindungsgemäße chirurgische Instrumente einen Fühler auf, der die Präsenz eines Druckgasspeichers in der Arbeitsstellung am Handgriff des Instruments prüft und optional auch anzeigt.

Bevorzugt weist das erfindungsgemäße chirurgische Instrument eine Tastvorrichtung auf, mit der das Einrücken des Druckgasspeichers erkannt werden kann.

Die Tastvorrichtung kann insbesondere mit einem mechanischen oder optischen Sensor ausgerüstet sein.

Die Abtastung kann auch am optionalen Druckspeichergehäuse oder einer Druckspeicher-Aufnahme, mit denen der Druckspeicher optional zusammen am Handgriff montiert wird, erfolgen.

Bevorzugt findet die Abtastung an dem Druckgasspeicher selbst statt. Hierbei kann die Abtastung entweder am Außenumfang des im Wesentlichen zylindrischen Druckgasspeichers erfolgen, an dessen Stirnseite oder gegebenenfalls im Bereich einer Schulter des sich typischerweise flaschenhalsartig verjüngenden Endes des Druckgasspeichers, mit dem dieser an dem Druckgaskanal des Instruments angeschlossen wird.

Als mechanische Sensoren kommen stiftartige Abtastelemente oder hohlzylindrische Elemente bevorzugt in Frage. Die stiftartigen Abtastelemente setzen auf einer punktuellen Oberfläche des Druckgasspeichers auf, sei es am Außenumfang, stirnseitig oder im Bereich der Schulter.

Die hohlzylindrischen Abtastelemente, die voll- oder teilzylindrisch ausgebildet sein können, setzen typischerweise auf einer Ringfläche im Bereich der Stirnseite oder der Schulter des Druckgasspeichers auf.

Stiftartige oder hohlzylindrische Abtastelemente lassen sich jedoch auch zum Abtasten eines gegebenenfalls verwendeten Druckgasspeichergehäuses, -halters, -hülse oder dergleichen verwenden.

Ebenso ist vorstellbar, dass die stiftartigen oder hohlzylindrischen Abtastelemente an einem Adapterelement angreifen, mit welchem der Druckgasspeicher versehen wird, bevor er am Handgriff an dem Druckgaskanal angeschlossen wird.

Darüber hinaus ist vorstellbar, dass die Abtastung an einem Verschlusselement eines Druckgasspeichergehäuses erfolgt, in das der Druckgasspeicher eingesetzt werden kann, bevor er an dem Druckgaskanal des Handgriffs angeschlossen wird.

Zur Verriegelung des Druckgasspeichers in seiner Arbeitsstellung wird vorzugsweise ein mechanisches Verriegelungselement verwendet.

Dieses rückt vorzugsweise in seiner aktiven Stellung in eine Ausnehmung am Druckgasspeicher selbst, an dessen Gehäuse oder sonstiger Halterung, einem Verschlusselement des Gehäuses bzw. der Halterung oder in einen Verriegelungsmechanismus für ein solches Gehäuse, eine solche Halterung oder des Verschlusselements ein.

Die Verriegelung über das mechanische Verriegelungselement wird vorzugsweise pneumatisch durch den von dem Druckgasspeicher gelieferten Gasdruck, weiter bevorzugt gegen die Kraft eines federelastischen Elements, bewirkt. Sobald der von dem Druckgasspeicher bereitgestellte Druck unter einen vorgegebenen Wert gefallen ist, wird das Verriegelungselement aus seiner aktiven Stellung in eine inaktive Stellung überführt und der Druckgasspeicher zur Entnahme freigegeben.

Weiter bevorzugt weist das chirurgische Instrument eine Steuerungsvorrichtung auf, welche bei einem vorgegebenen, ausreichend hohen Gasdruck in dem Druckgasspeicher die Funktion des Werkzeugs freigibt und bei einem zu geringen Gasdruck die Funktion des Werkzeugs sperrt. Solche erfindungsgemäßen chirurgischen Instrumente lassen sich insbesondere auch als Knochenstanzen ausbilden und einsetzen.

Diese und weitere Vorteile der Erfindung werden im Folgenden anhand der Zeichnung an einem bevorzugten Beispiel noch näher erläutert. Es zeigen im Einzelnen:
- Figur 1:: ein erfindungsgemäßes chirurgisches pneumatisch betriebenes Instrument in Form einer Knochenstanze in teilweise aufgebrochener Darstellung;
- Figur 2:: ein Detail der Knochenstanze der Figur 1;
- Figuren 3, 4 und 5:: eine Schnittdarstellung eines Teils der Knochenstanze der Figur 1 in unterschiedlichen Betriebszuständen; und
- Figuren 6A bis 6C:: Schaltbilder eines Teils der Pneumatik der Knochenstanze der Figur 1 zur Erläuterung der in den Figuren 3 bis 5 gezeigten Betriebszustände.

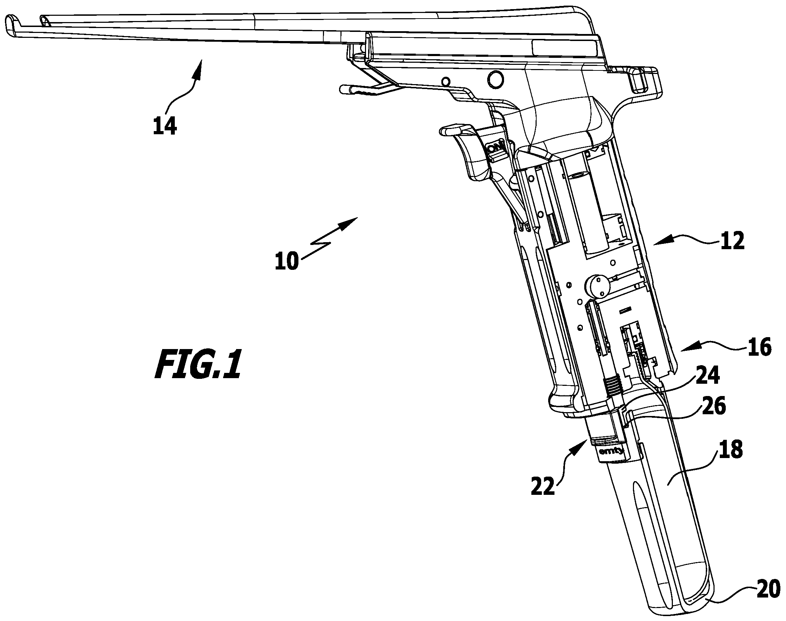

Figur 1 zeigt ein erfindungsgemäßes Instrument in Form einer Knochenstanze 10 mit einem pistolenförmig ausgebildeten Handgriff 12 und einem daran gehaltenen pneumatisch betreibbaren Stanzwerkzeug 14.

An seinem vom Werkzeug 14 beabstandeten Ende weist der Handgriff 12 eine Anschlussvorrichtung 16 auf, über die ein Druckgasspeicher 18 mit dem Handgriff 12 verbindbar ist. Der Druckgasspeicher 18 wird bei diesem Ausführungsbeispiel eines erfindungsgemäßen Instruments 10 zusammen mit einem hohlzylindrischen Gehäuseteil 20 am Handgriff 12 montiert, so dass die Verbindung zwischen dem Handgriff 12 bzw. dessen Druckgaskanal (hier nicht gezeigt) und dem Druckgasspeicher 18 gegen mechanische Einwirkungen geschützt ist.

In dem in Figur 1 gezeigten Betriebszustand ist der Druck in dem Druckgasspeicher 18 so weit abgefallen, dass mit dem in dem Druckgaskanal verfügbaren Druck ein sicheres Arbeiten mit dem Stanzwerkzeug 14 nicht mehr möglich ist und demzufolge die Funktion der Knochenstanze 10 blockiert ist. Um dem Personal den Grund anzuzeigen, der zur Blockierung des Stanzwerkzeugs 14 geführt hat, ist am Handgriff 12, benachbart zur Anschlussvorrichtung 16, ein Anzeigeelement 22 vorgesehen, dessen Schriftzug "empty" in diesem Betriebszustand freigegeben und damit sichtbar ist. Damit einher geht, dass ein Arretierelement 24 außer Eingriff mit einer Ausnehmung 26 am Gehäuseteil 20 steht und so das Gehäuseteil 20 mit dem Druckgasspeicher 18 vom Instrument durch Drehen des Bajonettverschlusses (im Einzelnen nicht gezeigt) getrennt werden kann.

Wie eingangs erwähnt, wird die Funktion der Knochenstanze 10 aus Sicherheitsgründen zu einem Zeitpunkt blockiert, zu dem noch ein erheblicher Restdruck in dem Druckgasspeicher 18 vorhanden ist. Um zu vermeiden, dass in diesem Zustand der Druckgasspeicher 18 ohne Weiteres von dem Handgriff 12 bzw. dessen Druckgaskanal getrennt werden kann, greift ein von dem Restdruck noch in einer aktivierten Stellung gehaltenes Arretierelement 24 in eine Ausnehmung 26 am oberen Umfang 28 des Gehäuseteils 20 ein.

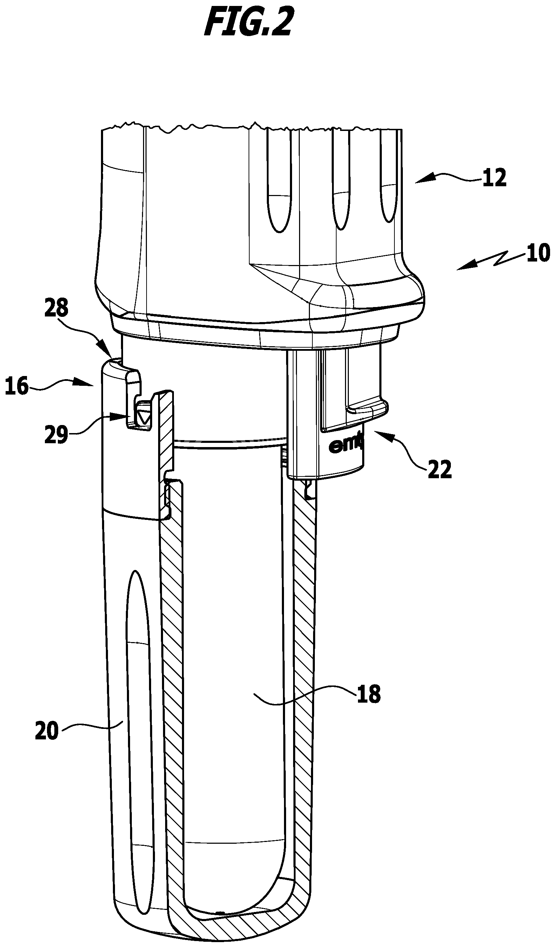

Dieser Betriebszustand wird im Folgenden anhand einer vergrößerten Detaildarstellung der Figur 2 noch näher erläutert.

Das Gehäuseteil 20 ist mit dem Handgriff 12 bzw. dessen Anschlussvorrichtung 16 im vorliegenden Ausführungsbeispiel mit einem Bajonettverschluss 29 gekoppelt, der an sich ein schnelles Auswechseln des Druckgasspeichers 18 erlaubt. Im gezeigten Betriebszustand der Figur 2 ist das Arretierelement 24 in einer inaktiven Stellung, in der es außer Eingriff mit der Ausnehmung 26 ist; der Restdruck in dem Druckgasspeicher 18 ist auf einen vorgegebenen Wert reduziert, der vorzugsweise im Wesentlichen dem Umgebungsdruck entspricht. Erst in diesem Zustand lässt sich der Bajonettverschluss mit einer Drehbewegung lösen, das Gehäuseteil 20 samt dem darin gehaltenen Druckgasspeicher 18 vom Handgriff 12 trennen und der Druckgasspeicher 18 letztlich austauschen.

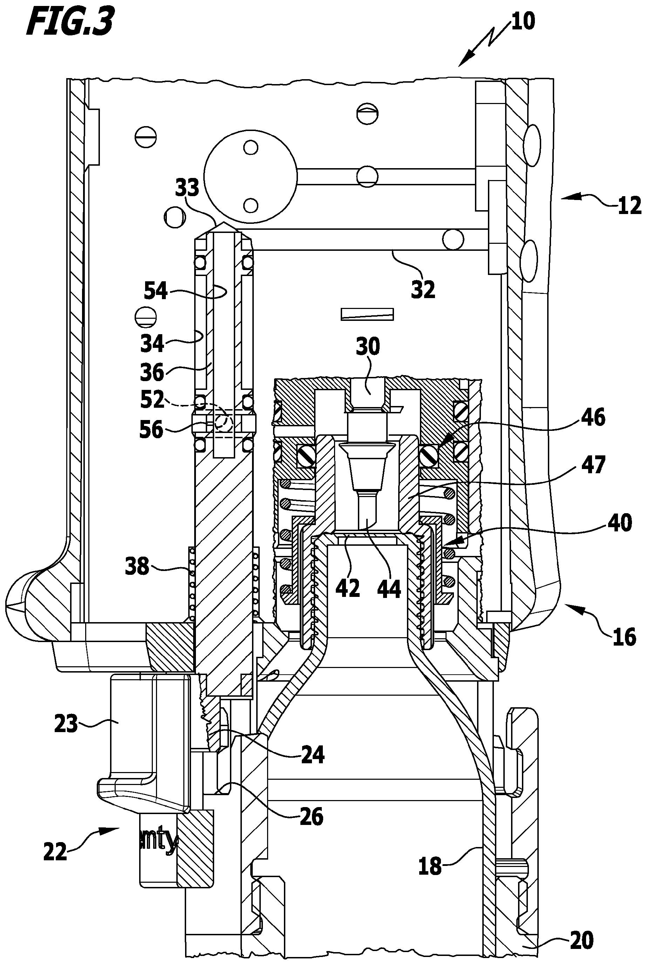

Anhand der Figuren 3 bis 5 soll im Folgenden der Ablauf beim Wechseln eines Druckgasspeichers 18 näher beschrieben werden.

Figur 3 zeigt eine Schnittdarstellung durch die Anschlussvorrichtung 16 des Handgriffs 12 in einem Betriebszustand, in dem ein frischer Druckgasspeicher 18 mit dem Instrument 10 verbunden werden soll. Der Druckgaskanal 30 des Handgriffs 12 ist in diesem Zustand drucklos. Demzufolge ist auch ein von dem Druckgaskanal 30 abzweigender Steuerungskanal 32 drucklos.

Der Steuerungskanal 32 ist mit dem geschlossenen Ende 33 einer zylindrischen Bohrung 34 in dem Handgriff 12 verbunden, in der längsverschieblich ein Steuerkolben 36 gelagert ist. Der Steuerkolben 36 wird mittels einer Schraubenfeder 38 im drucklosen Zustand des Steuerkanals 32 gegen das geschlossene Ende 33 der Bohrung 34 gedrückt. Das in dem vorliegenden Ausführungsbeispiel mit dem Steuerkolben 36 einstückig ausgebildete Arretierelement 24 befindet sich in der inaktiven Stellung.

Das Gehäuseteil 20 mit einem darin angeordneten frischen Druckgasspeicher oder Kartusche 18 kann jetzt mittels Bajonettverschluss mit der Anschlussvorrichtung 16 des Handgriffs 12 verbunden werden.

Die Kartusche 18 ist in dem in Figur 3 dargestellten Zustand an ihrem in die Anschlussvorrichtung eingeführten Ende 40 noch mit einer Versiegelung 42 gasdicht und druckfest verschlossen.

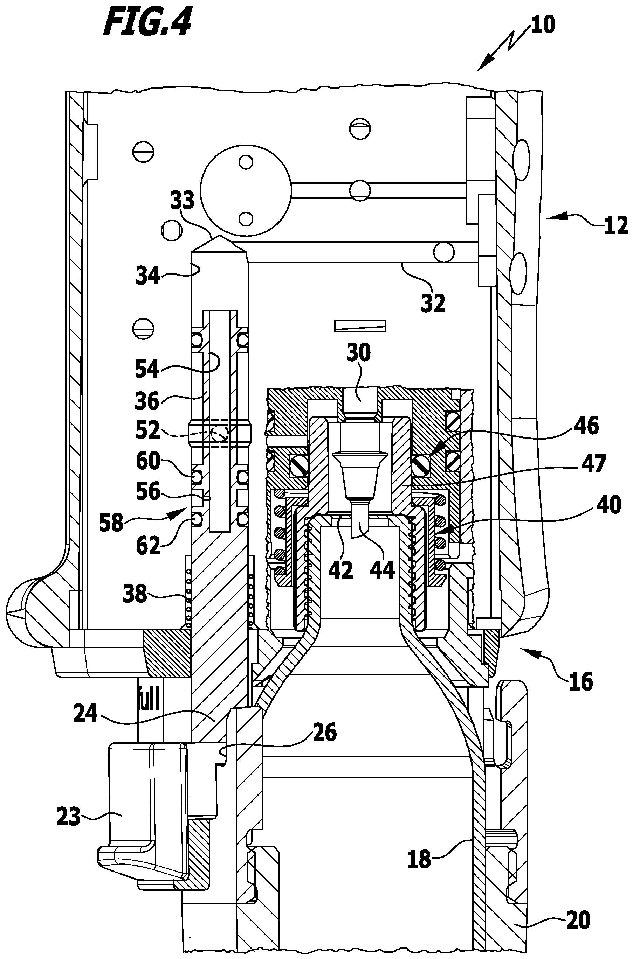

Der Druckgaskanal 30 des Handgriffs 12 endet im Bereich der Anschlussvorrichtung 16 mit einem zum Durchstechen der Versiegelung 42 gegen die Axialrichtung geneigt angeschliffenen hohlzylindrischen Ende 44. Die Steigung des Bajonettverschlusses ist so gewählt, dass mit der Drehung bei einer Schließbewegung das Gehäuseteil 20 die Kartusche 18 so weit in die Anschlussvorrichtung 16 des Handgriffs 12 einrückt, dass das Ende 44 die Versiegelung 42 vollständig durchsetzt und in das obere, offene Ende 40 der Kartusche 18 eindringt. Gleichzeitig wird der Außenumfang des oberen Endes 40 der Kartusche 18 oder, wie im vorliegenden Ausführungsbeispiel gezeigt, eine auf die Kartusche 18 aufgeschraubte Adaptierhülse 47 von einer in der Anschlussvorrichtung angeordneten Dichtung 46 erfasst, so dass eine gasdichte Verbindung zwischen der Kartusche 18 und dem Druckgaskanal 30 hergestellt ist. Diese Situation ist in Figur 4 dargestellt.

Der Druck der Kartusche 18, beispielsweise ca. 10 bis 12 bar, wirkt nun über den Druckgaskanal 30 und den Steuerkanal 32 auf das geschlossene Ende der Bohrung 34 und drückt den Steuerkolben 36 gegen die Feder 38 aus der inaktiven Stellung heraus. Das mit dem Steuerkolben 36 einstückig ausgebildete Arretierelement 24 rückt in die Ausnehmung 26 des Gehäuseteils 20 ein und blockiert den Bajonettverschluss. Das Instrument 10 ist jetzt betriebsbereit. Eine Trennung der Kartusche 18 von dem Handgriff 12 ist in diesem Zustand nicht möglich.

Fällt der Druck in der Gaskartusche 18 infolge der Betätigung des Instruments auf einen vorgegebenen Druckwert ab, beispielsweise ca. 2 bar, wird der Steuerkolben 36 in der Bohrung 34 mittels Federkraft in Richtung zum geschlossenen Ende 33 der Bohrung 34 bewegt. Das Arretierelement 24 bleibt jedoch in einer aktiven Stellung und greift, wenn auch nur partiell, in die Ausnehmung 26 des Gehäuseteils 20 ein.

In diesem Betriebszustand ist das Werkzeug in seiner Funktion blockiert und das Anzeigeelement steht zwischen den beiden Positionen "full" und "empty".

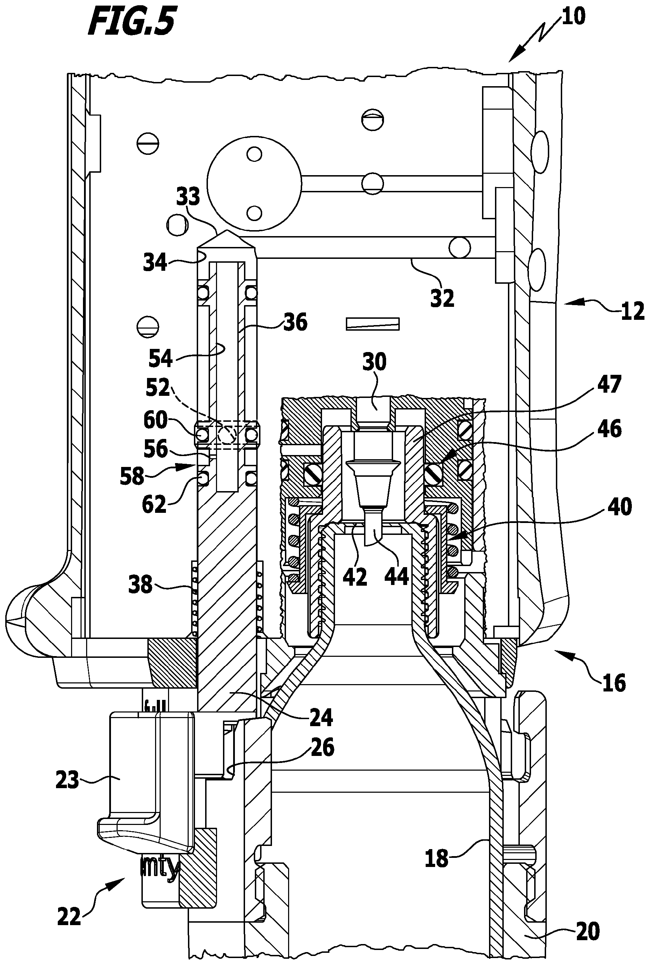

Mit dem an dem Arretierelement 24 angeordneten Schieber 23 lässt sich der Steuerkolben 36 in Richtung der Endposition (zweite Arbeitsstellung der Entriegelungsvorrichtung), wie in Figur 3 gezeigt, drücken, wobei schon in einer ersten Arbeitsstellung der Entriegelungsvorrichtung, die in Figur 5 gezeigt ist, eine Verbindung des Steuerkanals 32 und damit auch des Druckgaskanals 30 und des Gasvolumens der Kartusche 18 mit einer Entlüftungsleitung 52 hergestellt und aufrecht erhalten wird und der Restdruck der Gaskartusche 18 bis auf Umgebungsdruck abgebaut werden kann.

Hierzu weist der Steuerkolben 36 eine zentrale Axialbohrung 54 auf, die von dem Steuerkanal-seitigen Ende des Steuerkolbens 36 aus in Richtung zum Arretierelement 24 als Sackloch ausgebildet ist. Am geschlossenen Ende der Axialbohrung 54 ist eine Querbohrung 56 vorgesehen, die als Drossel ausgebildet ist.

Die Ausbildung der Querbohrung 56 als Drossel verhindert das Ausströmen größerer Druckgasmengen beim Einsetzen und Verbinden einer frischen Kartusche 18 mit dem Handgriff 12 und dessen Druckgaskanal 30.

Die Querbohrung 56 endet am Außenumfang des Steuerkolbens 36 in einem Abschnitt 58, der zu beiden Seiten in Axialrichtung gesehen über eine Dichtung 60 bzw. 62 gegen das Restvolumen der Bohrung 34 abgedichtet ist. Die Länge des Abschnitts 58 in Axialrichtung des Steuerkolbens 36 ist so bemessen, dass der Abschnitt 58 in Fließverbindung mit der Entlüftungsleitung 52 kommt, bevor das Arretierelement 24 vollständig aus der Ausnehmung 26 ausrückt. Damit kann eine kontrollierte Entlüftung des Druckgaskanals 30 sowie der Kartusche 18 eingeleitet werden, bevor das Gehäuseteil 20 mit dem Bajonettverschluss von dem Handgriff 12 getrennt werden kann.

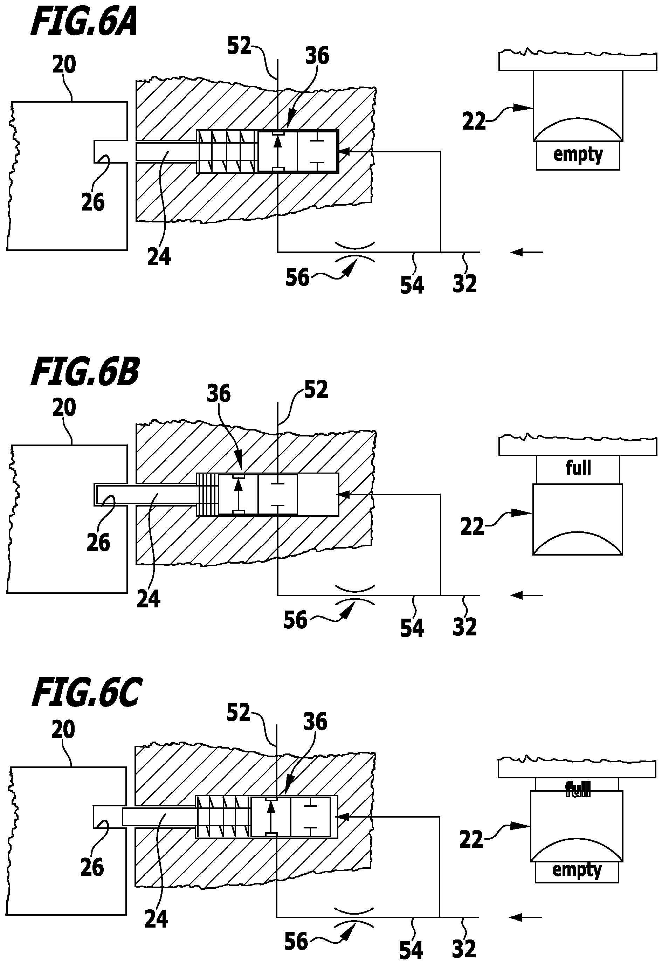

Zur Verdeutlichung der pneumatischen Schaltzustände der Figuren 3 bis 5 ist in den Figuren 6A bis 6C nochmals die Anzeigefunktion des Arretierelements 24 / Schiebers 23 zusammen mit einer Schaltskizze dargestellt, wobei die einzelnen Elemente dieselben Bezugszeichen tragen wie in den Figuren 3 bis 5 erläutert.

## Patentansprüche

1. Pneumatisch betätigbares chirurgisches Instrument (10), umfassend einen Handgriff (12) und ein an dem Handgriff (12) angeordnetes druckgasbetriebenes Werkzeug (14),
einen austauschbaren Druckgasspeicher (18),
sowie eine Verriegelungsvorrichtung (24;26) zum Festlegen des Druckgasspeichers an dem Handgriff (12) in einer Arbeitsstellung, in welcher der Druckgasspeicher (18) an einen Druckgaskanal (30) angeschlossen ist, mit dem das Werkzeug (14) mit Druckgas versorgt wird,
**dadurch gekennzeichnet, dass** das Instrument (10) ferner eine Entriegelungsvorrichtung (23) zum Aufheben der Festlegung des Druckgasspeichers (18) in der Arbeitsstellung umfasst, dass die Entriegelungsvorrichtung (23) eine Entlüftungsvorrichtung (36) aufweist, welche bei einer Betätigung der Entriegelungsvorrichtung (23) und deren Verbringung in eine erste Arbeitsstellung aus einer Ruhestellung in eine erste Arbeitsstellung bewegbar ist, in der der Druckgaskanal (30) mit einer Entlüftungsöffnung (56) verbunden ist, und dass die Entriegelungsvorrichtung (23) aus der ersten Arbeitsstellung in eine zweite Arbeitsstellung bewegbar ist, in der die Verriegelungsvorrichtung (24; 26) inaktiv und der Druckgasspeicher (18) zum Austausch freigegeben ist.

2. Chirurgisches Instrument (10) nach Anspruch 1, **dadurch gekennzeichnet, dass** das Instrument (10) einen Drucksensor zur Überwachung des Drucks in dem Druckgasspeicher (18) umfasst.

3. Chirurgisches Instrument (10) nach Anspruch 1 oder 2, **dadurch gekennzeichnet, dass** das Instrument (10) eine Anzeigevorrichtung (22) für den aktuellen Füllzustand des Druckgasspeichers (18) umfasst.

4. Chirurgisches Instrument (10) nach einem der Ansprüche 1 bis 3, **dadurch gekennzeichnet, dass** die Verriegelungsvorrichtung (24;26) und die Entriegelungsvorrichtung (23) als eine mechanische Einheit ausgebildet sind.

5. Chirurgisches Instrument (10) nach Anspruch 4, **dadurch gekennzeichnet, dass** die mechanische Einheit der Verriegelungs-und Entriegelungsvorrichtung eine Anzeigevorrichtung (22) des aktuellen Füllzustands des Druckgasspeichers (18) integriert umfasst.

6. Chirurgisches Instrument (10) nach einem der Ansprüche 1 bis 5, **dadurch gekennzeichnet, dass** die Entriegelungsvorrichtung (23) mit einem einzigen Betätigungselement aus der Ruhestellung in die erste Arbeitsstellung und die zweite Arbeitsstellung bewegbar ist.

7. Chirurgisches Instrument (10) nach einem der Ansprüche 1 bis 6, **dadurch gekennzeichnet, dass** das Instrument (10) einen Abluftkanal (52) aufweist, der verbrauchtes Druckgas aus dem Werkzeug (14) aufnimmt und in die Umgebung ableitet.

8. Chirurgisches Instrument (10) nach einem der Ansprüche 1 bis 7, **dadurch gekennzeichnet, dass** der Druckgaskanal (30) in der ersten Arbeitsstellung und in der zweiten Arbeitsstellung mit der Entlüftungsöffnung (56) in Fluidverbindung steht.

9. Chirurgisches Instrument (10) nach einem der Ansprüche 1 bis 8, **dadurch gekennzeichnet, dass** die Entlüftungsöffnung (56) oder der Abluftkanal (52) einen Schalldämpfer umfasst.

10. Chirurgisches Instrument (10) nach einem der Ansprüche 1 bis 9, **dadurch gekennzeichnet, dass** die Verriegelungsvorrichtung (20;24) pneumatisch, insbesondere durch das Druckgas (18) des Druckgasspeichers, aktivierbar ist.

11. Chirurgisches Instrument (10) nach einem der Ansprüche 1 bis 10, **dadurch gekennzeichnet, dass** der Druckgasspeicher (18) direkt am Handgriff (12) des Instruments (10) gehalten ist.

12. Chirurgisches Instrument (10) nach einem der Ansprüche 1 bis 10, **dadurch gekennzeichnet, dass** der Druckgasspeicher (18) in einem Gehäuse (20) am Handgriff (12) des Instruments (10) gehalten ist.

13. Chirurgisches Instrument (10) nach einem der Ansprüche 1 bis 12, **dadurch gekennzeichnet, dass** das Instrument (10) einen Fühler umfasst, der die Präsenz eines Druckgasspeichers (18) in der Arbeitsstellung am Handgriff (12) des Instruments (10) prüft und optional auch anzeigt.

14. Chirurgisches Instrument (10) nach einem der Ansprüche 1 bis 13, **dadurch gekennzeichnet, dass** das Instrument (10) eine Steuerungsvorrichtung umfasst, welche bei einem vorgegebenen, ausreichend hohen Gasdruck in dem Druckgaskanal (30) die Funktion des Werkzeugs (14) freigibt und bei einem zu geringen Gasdruck die Funktion des Werkzeugs (14) sperrt.

## Claims

1. A pneumatically actuatable surgical instrument (10) comprising a handle (12) and a pressurized gas-operated tool (14) arranged at the handle (12), a replaceable pressurized gas reservoir, and a locking device (24; 26) for fixing the pressurized gas reservoir (18) on the handle (12) in an operative position in which the pressurized gas reservoir is connected to a pressurized gas channel (30) by which the tool (14) is supplied with pressurized gas, **characterized in that** the instrument further comprises an unlocking device (23) for releasing the fixing of the pressurized gas reservoir (18) in the operative position, that the unlocking device (23) comprises a vent device (36) which upon actuation of the unlocking device (23) and upon bringing the unlocking device (23) in a first operative position is movable from a rest position to a first operative position in which the pressurized gas channel (30) is connected to a vent opening (56) and **in that** the unlocking device (23) is movable from the first operative position to a second operative position in which the locking device (24; 26) is inactive and the pressurized gas reservoir (18) is released for replacement.

2. The surgical instrument (10) in accordance with claim 1, **characterized in that** the instrument (10) comprises a pressure sensor for monitoring the pressure inside the pressurized gas reservoir (18).

3. The surgical instrument (10) in accordance with claim 1 or 2, **characterized in that** the instrument (10) comprises an indicating device (22) for indicating the current fill state of the pressurized gas reservoir (18).

4. The surgical instrument (10) in accordance with any of claims 1 to 3, **characterized in that** the locking device (24; 26) and the unlocking device (23) are configured as one mechanical unit.

5. The surgical instrument (10) in accordance with claim 4, **characterized in that** the mechanical unit of the locking device and the unlocking device comprises integrated therein an indicating device (22) for indicating the current fill state of the pressurized gas reservoir (18).

6. The surgical instrument (10) in accordance with any of claims 1 to 5, **characterized in that** the unlocking device (23) is movable from the rest position to the first operative position and the second operative position by use of a single actuating element.

7. The surgical instrument (10) in accordance with any of claims 1 to 6, **characterized in that** the instrument (10) comprises an exhaust channel (52) that receives spent pressurized gas from the tool (14) and exhausts same to the environment.

8. The surgical instrument (10) in accordance with any of claims 1 to 7, **characterized in that** the pressurized gas channel (30) is in fluid communication with the vent opening (56) when in the first operative position and in the second operative position.

9. The surgical instrument (10) in accordance with any of claims 1 to 8, **characterized in that** the vent opening (56) or the exhaust channel (52) comprises a silencer.

10. The surgical instrument (10) in accordance any of claims 1 to 9, **characterized in that** the locking device (24; 26) is pneumatically activatable, in particular by the pressurized gas of the pressurized gas reservoir (18).

11. The surgical instrument (10) in accordance with any of claims 1 to 10, **characterized in that** the pressurized gas reservoir (18) is held at the handle (12) of the instrument (10) directly.

12. The surgical instrument (10) in accordance with any of claims 1 to 10, **characterized in that** the pressurized gas reservoir (18) is held in a housing (20) at the handle (12) of the instrument (10).

13. The surgical instrument (10) in accordance with any of claims 1 to 12, **characterized in that** the instrument (10) comprises a sensor which checks for and optionally also indicates the presence of a pressurized gas reservoir (18) in the operative position at the handle (12) of the instrument (10).

14. The surgical instrument (10) in accordance with any of claims 1 to 13, **characterized in that** the instrument (10) comprises a control device which enables the function of the tool (14) when the gas pressure inside the pressurized gas channel (30) is at a predetermined, sufficient level and disables the function of the tool (14) when the gas pressure is too low.

## Revendications

1. Instrument chirurgical (10) à actionnement pneumatique, comprenant
une poignée (12) et un outil (14) agencé sur la poignée (12) et fonctionnant à l'aide d'un gaz sous pression,
un accumulateur de gaz sous pression (18), interchangeable,
ainsi qu'un dispositif de verrouillage (24; 26) pour fixer l'accumulateur de gaz sous pression à la poignée (12) dans une position de travail dans laquelle l'accumulateur de gaz sous pression (18) est raccordé à un canal de gaz sous pression (30) à l'aide duquel l'outil (14) est alimenté en gaz sous pression,
**caractérisé en ce que** l'instrument (10) comprend, par ailleurs, un dispositif de déverrouillage (23) destiné à débloquer la fixation de l'accumulateur de gaz sous pression (18) dans la position de travail, **en ce que** le dispositif de déverrouillage (23) comprend un dispositif de purge (36), qui, lors d'un actionnement du dispositif de déverrouillage (23) et de son amenée dans une première position de travail, peut être déplacé d'une position de repos à une première position de travail dans laquelle le canal de gaz sous pression (30) est relié à une ouverture de purge (56), et **en ce que** le dispositif de déverrouillage (23) peut être déplacé de la première position de travail à une deuxième position de travail dans laquelle le dispositif de verrouillage (24; 26) est inactif et l'accumulateur de gaz sous pression (18) est libéré pour être échangé.

2. Instrument chirurgical (10) selon la revendication 1, **caractérisé en ce que** l'instrument (10) comprend un capteur de pression pour surveiller la pression dans l'accumulateur de gaz sous pression (18).

3. Instrument chirurgical (10) selon la revendication 1 ou la revendication 2, **caractérisé en ce que** l'instrument (10) comporte un dispositif de visualisation (22) pour le niveau de remplissage actuel de l'accumulateur de gaz sous pression (18).

4. Instrument chirurgical (10) selon l'une des revendications 1 à 3, **caractérisé en ce que** le dispositif de verrouillage (24; 26) et le dispositif de déverrouillage (23) sont réalisés sous la forme d'une unité mécanique.

5. Instrument chirurgical (10) selon la revendication 4, **caractérisé en ce que** l'unité mécanique du dispositif de verrouillage et de déverrouillage comprend de manière intégrée, un dispositif de visualisation (22) du niveau de remplissage actuel de l'accumulateur de gaz sous pression (18).

6. Instrument chirurgical (10) selon l'une des revendications 1 à 5, **caractérisé en ce que** le dispositif de déverrouillage (23) peut être déplacé à l'aide d'un seul élément d'actionnement, de la position de repos à la première position de travail et à la deuxième position de travail.

7. Instrument chirurgical (10) selon l'une des revendications 1 à 6, **caractérisé en ce que** l'instrument (10) présente un canal d'échappement d'air (52), qui collecte du gaz sous pression utilisé de l'outil (14), et l'évacue dans l'environnement.

8. Instrument chirurgical (10) selon l'une des revendications 1 à 7, **caractérisé en ce que** le canal de gaz sous pression (30) est en liaison fluidique avec l'ouverture de purge (56), dans la première position de travail et dans la deuxième position de travail.

9. Instrument chirurgical (10) selon l'une des revendications 1 à 8, **caractérisé en ce que** l'ouverture de purge (56) ou le canal d'échappement d'air (52) comporte un atténuateur de bruit.

10. Instrument chirurgical (10) selon l'une des revendications 1 à 9, **caractérisé en ce que** le dispositif de verrouillage (24; 26) peut être activé par voie pneumatique, notamment par le gaz sous pression de l'accumulateur de gaz sous pression (18).

11. Instrument chirurgical (10) selon l'une des revendications 1 à 10, **caractérisé en ce que** l'accumulateur de gaz sous pression (18) est maintenu directement sur la poignée (12) de l'instrument (10).

12. Instrument chirurgical (10) selon l'une des revendications 1 à 10, **caractérisé en ce que** l'accumulateur de gaz sous pression (18) est maintenu dans un boitier (20) sur la poignée (12) de l'instrument (10) .

13. Instrument chirurgical (10) selon l'une des revendications 1 à 12, **caractérisé en ce que** l'instrument (10) comporte une sonde qui vérifie et, en option, visualise également la présence d'un accumulateur de gaz sous pression (18) dans la position de travail sur la poignée (12) de l'instrument (10).

14. Instrument chirurgical (10) selon l'une des revendications 1 à 13, **caractérisé en ce que** l'instrument (10) comprend un dispositif de commande, qui, pour une pression de gaz prédéterminée, suffisamment élevée, dans le canal de gaz sous pression (30), valide le fonctionnement de l'outil (14), et pour une pression de gaz trop faible, bloque le fonctionnement de l'outil (14).
